(19)

**Europäisches Patentamt**

**European Patent Office**

**Office européen des brevets**

(11) **EP 4 760 658 A1**

(12) **EUROPEAN PATENT APPLICATION**

(43) Date of publication:
**17.06.2026 Bulletin 2026/25**

(21) Application number: **25219089.7**

(22) Date of filing: **27.11.2025**

(51) International Patent Classification (IPC):
**G06V 20/59** *(2022.01)* **G06V 10/82** *(2022.01)*
**G06V 10/25** *(2022.01)* **G06V 10/774** *(2022.01)*
**G06V 10/776** *(2022.01)*

(52) Cooperative Patent Classification (CPC):
**G06V 20/597; G06V 10/62; G06V 10/82;**
G06F 2218/00; G06V 10/25; G06V 10/774;
G06V 10/776

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **11.12.2024 US 202418977607**

(71) Applicant: **Harman Becker Automotive Systems,
Inc.
Novi, MI 48377 (US)**

(72) Inventors:
- **KOLODZIEJCZYK, Waldemar Jan
Novi, MI, 48377 (US)**
- **HARIHARAKRISHNAN, Anilkumar
Novi, MI, 48377 (US)**

(74) Representative: **Westphal, Mussgnug & Partner,
Patentanwälte mbB
Werinherstraße 79
81541 München (DE)**

(54) **SYSTEMS AND METHODS FOR RESPIRATION CHARACTERISTIC ESTIMATION**

(57) Systems and methods are herein provided for in-vehicle respiratory characteristic estimation. In one example, a vehicle computing system of a vehicle comprises one or more processors; memory storing instructions that when executed cause the one or more processors to: obtain video data comprising a plurality of frames from one or more visual sensing devices within the vehicle; extract motion data from the video data; determine, from the motion data, a breathing wave and one or more respiratory characteristics of a subject within the vehicle; and output the breathing wave and one or more respiratory characteristics to one or more of an advanced driver assistance system (ADAS) and a health and wellbeing system.

EP 4 760 658 A1

**Description**

FIELD

The present disclosure relates generally to driver state biosignals monitoring, and more particularly to respiration characteristic estimation.

BACKGROUND

Developments in the field of autonomous driving tackle issues regarding driver safety and comfort, personalization of the driving experience to individual users, and monitoring of the health and wellbeing of users. Health monitoring is often based on the time-resolved detection of biosignals, which act as measures of versatile electrical or non-electrical signals of the human body. such biosignals include, for example, heart beat features (e.g., pulse, interbeat intervals, pulse waves, etc.), respiration (breathing wave, breathing rate, breathing rate variability, breathing phase status, etc.), eye and eye-lid related information (gaze direction, eye openness), body agility, muscle tonus, body temperature, blood pressure, blood oxygen saturation, skin conductance, and many more. Determining such information about the driver can be provided to vehicle systems, such as autonomous driving systems or advances driver assistance systems (ADAS) to improve their functionality and thereby improve the driving experience.

However, determining respiration characteristics based upon in-vehicle signals, such as camera signals, is difficult with current technologies due to noise, changes in lighting, visibility, and the computational effort to analyze camera signals. More invasive efforts may be more reliable but are generally unsuited to vehicle applications. Further, respiratory characterizations can also be prone to inaccuracies due to external factors such as driver movement (e.g., shifting in seat, sighing, turning the steering wheel), road surface imperfections (e.g., bumpy roads), and the like. Further factors such as clothing variability, which may cause varying levels of reflection, presence and usage of seat based massage, pneumatic devices like portable oxygen concentrators, and presence of other passengers in the car that may cause the driver to move or may cause movement that is detected by sensors, may also result in inaccurate estimations.

Further still, human behavior and activities which demand flow of air through the respiratory system can influence respiratory characteristics, making it more difficult for standard sensors and methods to accurately determine respiratory characteristics. For example, speech and speech like activities can be difficult to separate from regular breathing as the respiratory system in the human body functions differently while speaking vs not-speaking. Other actions, like coughing or sneezing may disrupt regular breathing and can introduce variability in the data. Additionally, certain chronic conditions like asthma, chronic obstructive pulmonary disease, and chronic respiratory failure can present differently than a healthy person, rendering current methods for determining respiratory characteristics difficult for drivers with such conditions.

SUMMARY

In one or more embodiments, a vehicle computing system of a vehicle comprises one or more processors; memory storing instructions that when executed cause the one or more processors to: obtain video data comprising a plurality of frames from one or more visual sensing devices within the vehicle; extract motion data from the video data; determine, from the motion data, a breathing wave and one or more respiratory characteristics of a subject within the vehicle; and output the breathing wave and one or more respiratory characteristics to one or more of an advanced driver assistance system (ADAS) and a health and wellbeing system.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

BRIEF DESCRIPTION OF THE DRAWINGS

The disclosure may be better understood from reading the following description of non-limiting embodiments, with reference to the attached drawings, wherein below:

FIG. 1 shows an exemplary vehicle system including a respiratory characteristic determination model, in accordance with one or more embodiments of the present disclosure;
FIG. 2 shows an example partial view of a vehicle cabin including an instrument panel, in accordance with one or more embodiments of the present disclosure;
FIG. 3 shows a block diagram of an example in-vehicle computing system of a vehicle, in accordance with one or more embodiments of the present disclosure;

FIG. 4 shows a block diagram of a respiratory characteristic estimation model, in accordance with one or more embodiments of the present disclosure;

FIG. 5 shows a flowchart illustrating a high-level method for determining respiratory characteristics and estimated uncertainty thereof, in accordance with one or more embodiments of the present disclosure;

FIG. 6 shows a flowchart illustrating a method for determining respiratory characteristics with a respiratory characteristic estimation model, in accordance with one or more embodiments of the present disclosure;

FIG. 7 shows a flowchart illustrating a method for training the respiratory characteristic estimation model, in accordance with one or more embodiments of the present disclosure;

FIG. 8 shows a flowchart illustrating a method for determining an uncertainty estimation in respiratory characteristics, in accordance with one or more embodiments of the present disclosure; and

FIG. 9 shows a graph illustrating a trend of respiratory characteristics as outputted by a respiratory characteristic estimation model, in accordance with one or more embodiments of the present disclosure.

## DETAILED DESCRIPTION

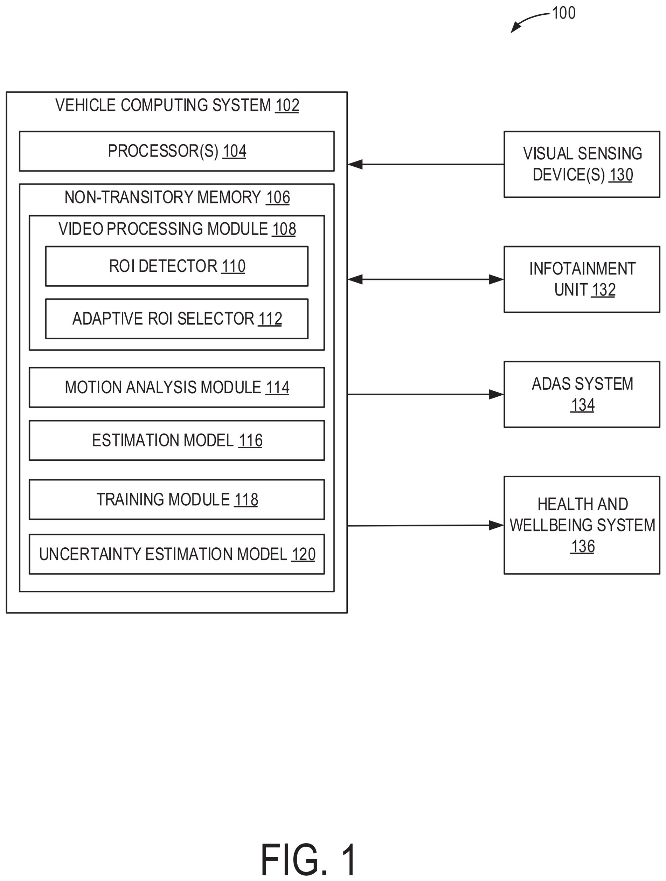

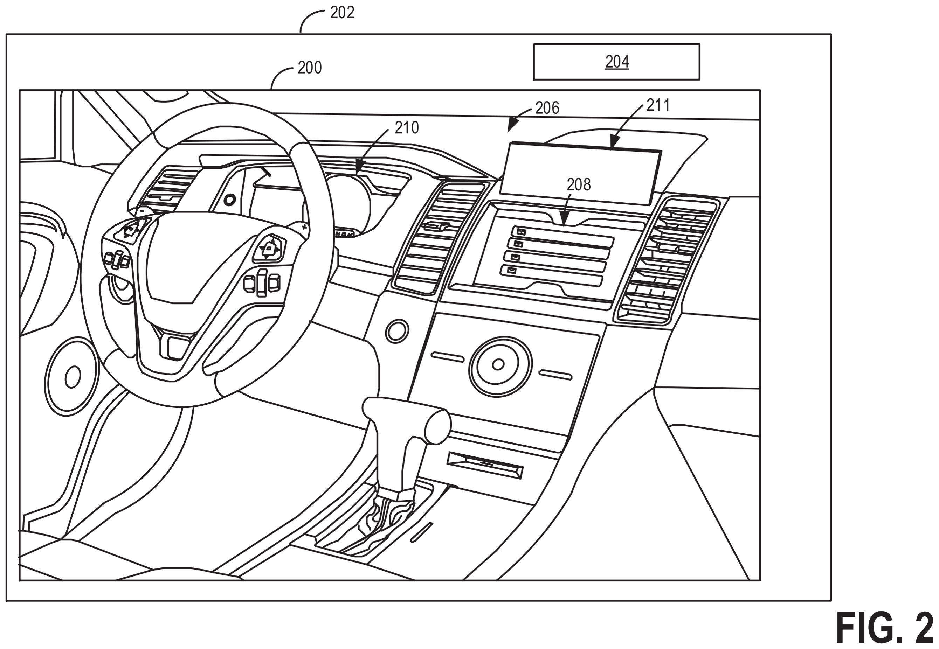

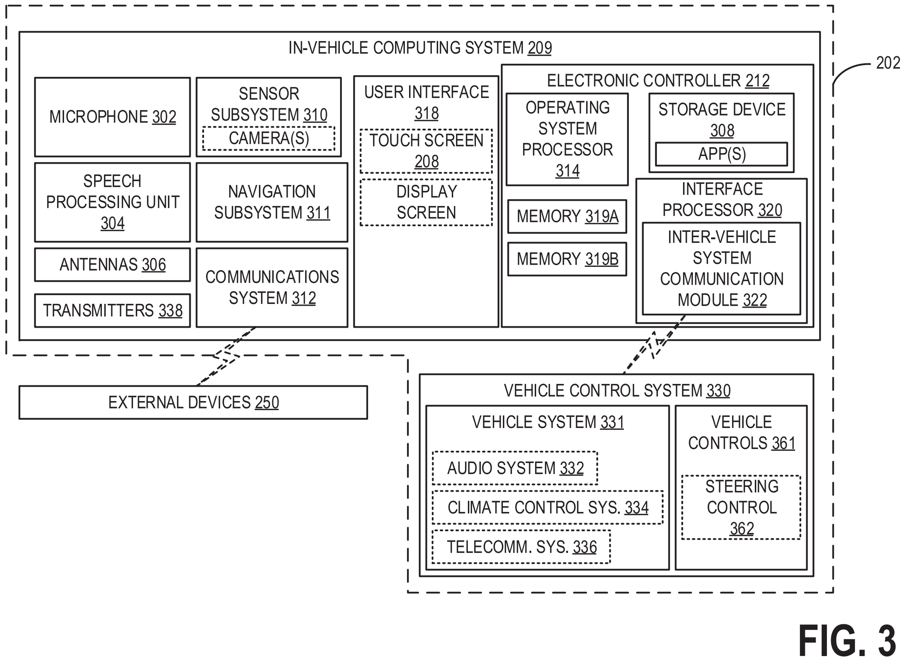

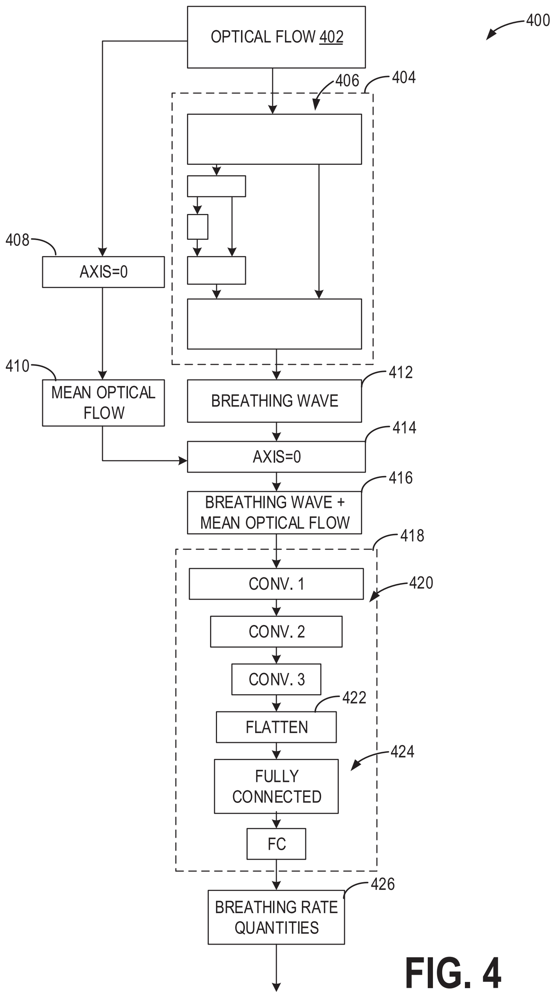

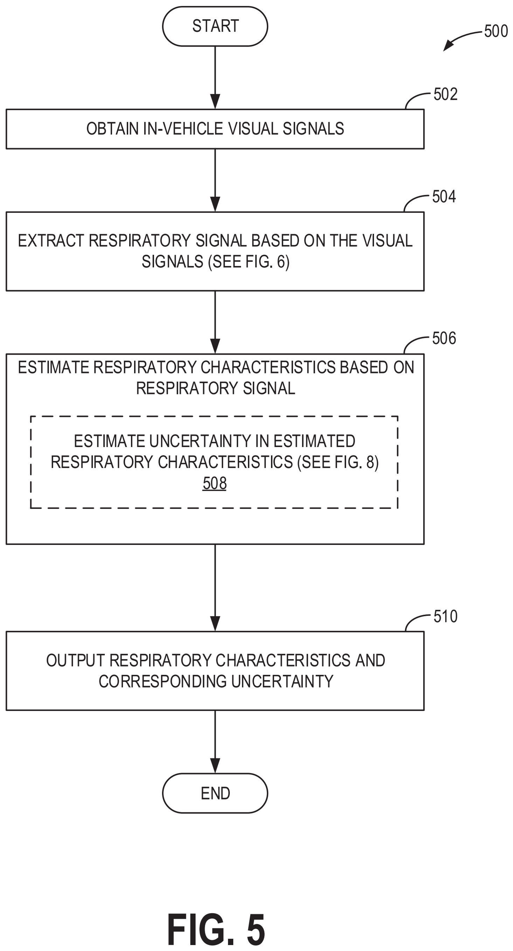

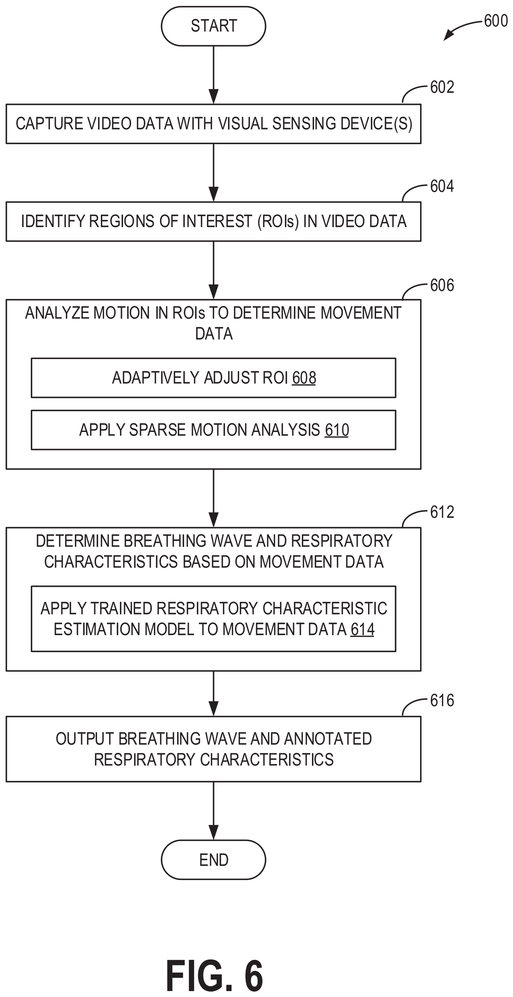

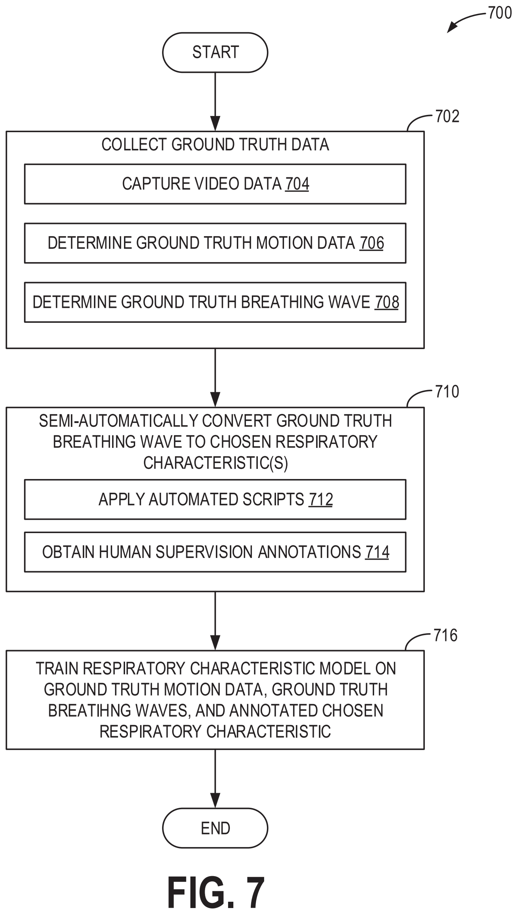

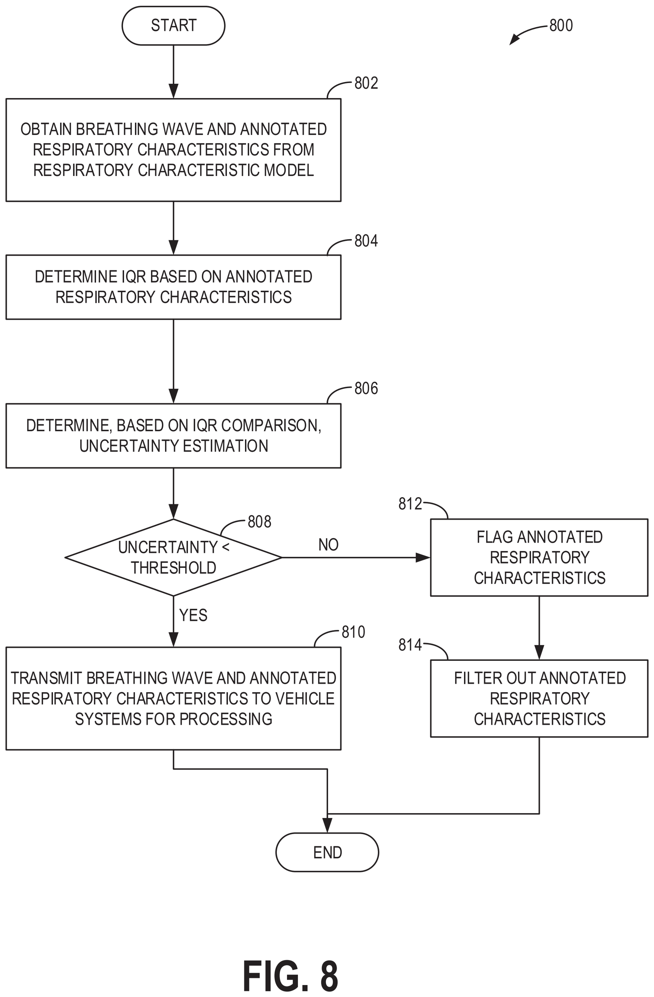

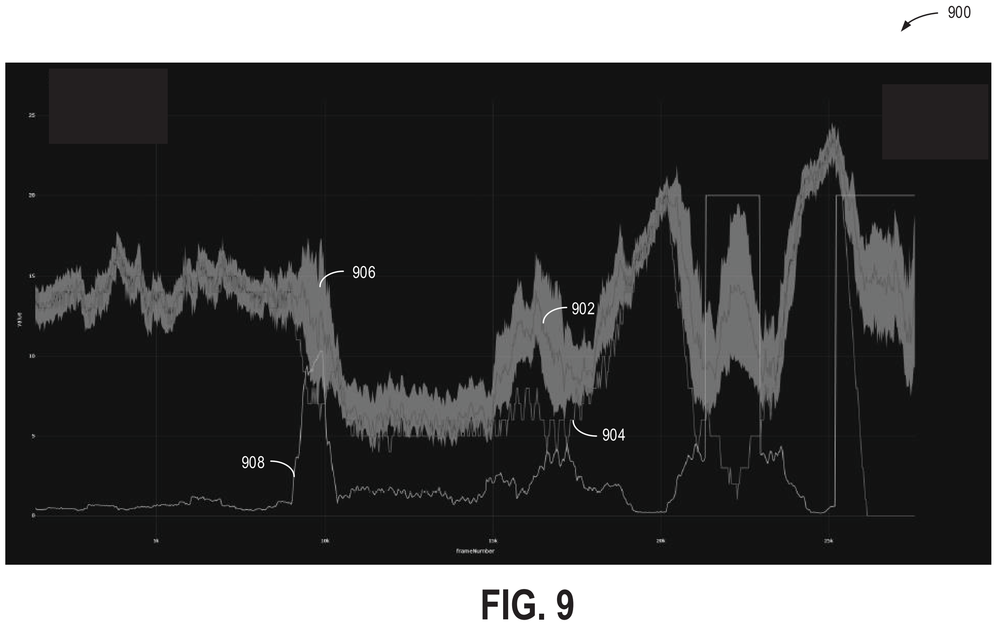

The following description relates to systems and methods for estimating respiratory characteristics of a driver in a vehicle system. In particular, systems and methods for determining respiratory characteristics from obtained in-vehicle visual signals and for estimating uncertainty in the determined respiratory characteristics is herein presented. Respiratory characteristics may be determined using a specifically trained respiratory characteristic estimation model, in some examples. The respiratory characteristic estimation model, and an uncertainty estimation model, may be incorporated into a vehicle computing system of a vehicle that is configured to ingest biosignals acquired by in-vehicle devices such as cameras. FIG. 1 shows an example vehicle system. FIG. 2 shows an example partial view of a vehicle cabin. FIG. 3 shows a block diagram of an example in-vehicle computing system of a vehicle system, such as the vehicle system of FIG. 1. FIG. 4 shows a block diagram of an exemplary respiratory characteristic estimation model that is incorporated into the vehicle computing system. FIG. 5 is a flowchart illustrating a high-level method for estimating respiratory characteristics and uncertainty thereof. FIG. 6 shows a flowchart illustrating a method for determining respiratory characteristics with a trained respiratory characteristic estimation model and FIG. 7 shows a flowchart illustrating a method for training the respiratory characteristic estimation model. FIG. 8 shows a flowchart illustrating a method for estimating uncertainty in an output of the respiratory characteristic estimation model. FIG. 9 shows a graph showing breathing wave trends, including a trend as outputted by the respiratory characteristic estimation model, a ground truth trend, and uncertainty parameter trend.

FIG. 1 shows a vehicle system 100, including a vehicle computing system 102. The vehicle system 100 as herein presented may be a vehicle configured for driving on roads, such as a battery electric vehicle (BEV), a hybrid electric vehicle (HEV, vehicle with an internal combustion engine, or any other type of suitable vehicle. Vehicle computing system 102 includes one or more processors 104 configured to execute machine readable instructions stored in non-transitory memory 106. Memory 106 and other memory referred to herein may include one or more data storage structures, such as optical memory devices, magnetic memory devices, or solid-state memory devices, for storing programs and routines executed by processor(s) 104 to carry out various functionalities disclosed herein. Memory 106 may include any desired type of volatile and/or non-volatile memory such as, for example, static random access memory (SRAM), dynamic random access memory (DRAM), flash memory, read-only memory (ROM), etc.

Processor(s) 104 and other processors referred to herein may be any suitable processor, processing unit, or microprocessor configured for use in a vehicle application, for example. Processor(s) 104 may be a multi-processor system, and thus, may include one or more additional processors that are identical or similar to each other and that are communicatively coupled via an interconnection bus. Processor(s) may be single core or multi-core, and the programs executed thereon may be configured for parallel or distributed processing. In some embodiments, processor(s) 104 may optimally include individual components that are distributed throughout two or more devices, which may be remotely located and/or configured for coordinated processing. In some embodiments, one or more aspects of processor(s) 104 may be virtualized and executed by remotely-accessible networked computing devices configured in a cloud computing configuration.

The vehicle system 100 may also comprise one or more visual sensing devices 130, an infotainment unit 132, an advanced driver assistance system 134, and a health and wellbeing system 136. The one or more visual sensing devices 130 may be configured for acquiring visual signals of a user of the vehicle system 100 (e.g., a driver) and about the vehicle. For example, the one or more visual sensing devices 130 may be near-infrared cameras (NICs) configured to capture near-infrared images of the driver of the vehicle. In some examples, the one or more visual sensing devices 130 may be configured to capture visual signals at predefined intervals (e.g., at a given framerate) such as 24 frames per second, 30 frames per second, 48 frames per second, or the like. It should be understood that other types of vehicle sensors, such as telemetry sensors that indicate position within a lane, steering wheel position, pedal position, speed, acceleration, yaw rate, and the like may also be included in the vehicle system 100.

The infotainment unit 132, as will be further described with respect to FIG. 3, may include a display device, a user interface, and various subsystems for displaying data such as audio data, like music, navigation notifications, etc. and visual data, like media and text notifications, navigation supplies, and the like. The ADAS system 134 may be configured for various vehicle operation functions, including adaptive cruise control, lane assistance, autonomous driving mode, collision warnings, automatic braking, blind spot detection, intelligent speed assistance, pedestrian detection, and the like using a combination of sensors including external facing cameras, radar, light detection and ranging (LiDAR), and/or the like. The health and wellbeing system 136 may be configured to ingest data from a plurality of sensing systems and construct a profile of a given driver based on the data. For example, the health and wellbeing system 136 may be configured to ingest information about a driver's health status (e.g., heartrate, respiration, and the like) as well as their emotional/cognitive state. Thus, the estimated respiratory characteristics, as determined by the vehicle computing system 102, may be transmitted to the health and wellbeing system 136 for further processing and/or storage. In some examples, the health and wellbeing system 136 may be integrated into the vehicle computing system 102.

The non-transitory memory 106, for determination of estimated respiratory characteristics and estimated uncertainty therein, may include one or more models and/or modules storing instructions executable by the processor(s) 104. For example, the non-transitory memory 106 may include a video processing module 108, a motion analysis module 114, a respiratory characteristic estimation model 116, a model training module 118, and an uncertainty estimation model 120.

The video processing module 108 may be configured to ingest visual data captured by the visual sensing device 130. A region of interest (ROI) detector 110 may be configured to detect particular ROIs within the visual signals, such as the driver's face, chest, and/or abdomen. The ROI detector 110 may identify and isolate these ROIs. An adaptive ROI selector 112 may be configured to dynamically adjust the ROIs based on the driver's movements, posture changes, and the like. For example, the visual signal data captured by the visual sensing device 130 may be captured iteratively at a predefined framerate. An ROI may first be identified and selected in a first frame by the ROI detector 110 and then the ROI may be dynamically adjust the position of the ROI within subsequent frames based on the driver's movements by the adaptive ROI selector 112. In this way, the ROI detector 110 may only need to detect the ROIs one time per session, thereby reducing overall processing demands.

The motion analysis module 114 may store instructions for tracking specific points of interest, for example within the identified ROIs, across time (e.g., across video frames). As an example, the motion analysis module 114 may employ a sparse motion analysis technique to the ROI such as an optical flow motion algorithm. As an example, an optical flow motion technique may comprise determining frame to frame motion vectors of one or more designated points and/or the small patches around the designated points. The output of the sparse motion analysis/optical flow algorithm may be a motion field comprising displacement vectors of the designated points.

The respiratory characteristic estimation model 116 may be configured to take as input the output of the motion analysis (e.g., the motion vector field) and determine estimated respiratory characteristics. As will be further explained below, the respiratory characteristic estimation model 116 may be specifically trained on ground truth data obtained in vehicle settings or simulated vehicle settings. For example, breathing wave ground truth may be semi-automatically converted to respiratory characteristics using a combination of automated scripts and human supervision. The respiratory characteristic estimation model 116 may be an artificial intelligence (AI) model, such as a deep neural network (DNN), a convolutional neural network (CNN), or the like. An example diagram of the respiratory characteristic estimation model 116 is shown in FIG. 4. The training module 118 may be configured to train the respiratory characteristic estimation model 116.

The uncertainty estimation model 120 may be configured to estimate uncertainty in an output of the respiratory characteristic estimation model 116. For example, the respiratory characteristic estimation model 116 may output a breathing wave and one or more associated characteristics, such as breathing rate and breathing rate variability. The one or more associated characteristics may be returned in quantities. For example, breathing rate may be returned by the model as the $10^{th}$ percentile of breathing rate (the value of breathing rate below which, on average, lay 10% of possible outcomes for similar inputs), $50^{th}$ percentile of breathing rate (to serve as a main breathing rate prediction), and $90^{th}$ percentile of breathing rate (the value of breathing rate below which, on average, lay 90% of possible outcomes for similar inputs). The uncertainty estimation model 120 may ingest these quantities and determine, based on interquartile range (IQR) and comparison of IQR against one or more reliability parameters like absolute error, as will be further described with respect to FIG. 8, an estimation of the uncertainty of the respiratory characteristics as outputted by the respiratory characteristic estimation model.

FIG. 2 shows an example partial view of an interior of a cabin 200 of a vehicle 202, in which a driver and/or one or more passengers may be seated. Vehicle 202 may be the vehicle system 100 shown by FIG. 1 and described above, in some examples.

Vehicle 202 of FIG. 2 may be a motor vehicle including drive wheels (not shown) and a power source 204 configured to provide torque to the drive wheels, such as an internal combustion engine and/or battery. In examples in which the power source 204 includes an internal combustion engine, the internal combustion engine may include one or more combustion chambers which may receive intake air via an intake passage and exhaust combustion gases via an exhaust passage. Vehicle 202 may be a road automobile, among other types of vehicles. In some examples, vehicle 202 may include a hybrid propulsion system including an energy conversion device operably to absorb energy from vehicle motion and/or the engine and convert the absorbed energy to an energy form suitable for storage by an energy storage device. Vehicle 202 may be a fully electric vehicle in some examples, incorporating vehicle cells, solar energy capturing elements, and/or other energy storage systems for powering the vehicle.

As shown, the vehicle 202 may include an instrument panel 206 with various displays and controls accessible to a human driver (also referred to as the user and/or occupant) of the vehicle 202. For example, instrument panel 206 may include a touch screen 208 of an in-vehicle computing system (e.g., vehicle computing system 102 of FIG. 1) and an instrument cluster 210. Touch screen 208 may receive user input to the in-vehicle computing system for controlling visual display output, user preferences, control parameter selection, and so on. While the example system shown in FIG. 2 includes controls that may be performed via a user interface in the in-vehicle computing system, such as touch screen 208, without a separate control panel, in other embodiments, the vehicle may include additional control panels. In some embodiments, one or more hardware elements of in-vehicle computing system 209, such as touch screen 208, a display screen 211 (e.g., display screen 116), various control dials, knobs and buttons, memory, processor(s), and my interface elements (e.g., connectors or ports) may form an integrated head unit that is installed in instrument panel 206 of the vehicle. The head unit may be fixedly or removably attached in instrument panel 206. In additional or alternative embodiments, one or more hardware elements of in-vehicle computing system 209 may be modular and may be installed in multiple locations of the vehicle.

During operation of the vehicle 202, the in-vehicle computing system may be configured to receive electronic signals from the various sensors of the vehicle 202, such as the visual sensing device 130 of FIG. 1, in some examples. The signals from the various sensors may be decoded, translated, or otherwise processed by the in-vehicle computing system in order to determine estimated respiratory characteristics and/or uncertainty. It should be understood that other sensors may also provide signals to the in-vehicle computing system and the in-vehicle computing system may process those signals to perform other functions, such as to determine heart rate, mental state (e.g., anxiety level, cognitive load, etc.), and the like.

FIG. 3 shows a block diagram of an in-vehicle computing system 209 integrated inside vehicle 202, wherein in-vehicle computing system 209 may be a non-limiting example of vehicle computing system 102 of vehicle system 100 of FIG. 1. In-vehicle computing system 209 may be referred to herein as a controller and/or electronic controller in some examples. In-vehicle computing system 209 may perform one or more of the methods described herein in some embodiments. In-vehicle computing system 209 may include, or be coupled to, various vehicle systems, sub-systems, hardware components, as well as software applications and systems that are integrated in, or integratable into, vehicle 202.

In-vehicle computing systems 209 may include one or more processors including an operating system processor 314 and an interface processor 320. Operating system processor 314 may execute an operating system on in-vehicle computing system 209, and control input/output, display, and other operations of in-vehicle computing system 209. Interface processor 320 may communicate with a vehicle control system 330 via an inter-vehicle system communication module 322.

Inter-vehicle system communication module 322 may output data to one or more other vehicle systems 331 and/or one or more other vehicle control elements 361, while also receiving data input from other vehicle systems 331 and other vehicle control elements 361, e.g., by way of vehicle control system 330. When outputting data, inter-vehicle system communication module 322 may provide a signal via a bus corresponding to any status of the vehicle, the vehicle surroundings, or the output of any other information source connected to the vehicle. Vehicle data outputs may include, for example, analog signals (such as current velocity), digital signals provided by individual information sources (such as clocks, thermometers, location sensors such as GPS sensors, and so on), digital signals propagated through vehicle data networks (such as an engine controller area network (CAN) bus through which engine related information may be communicated, a climate control CAN bus through which climate control related information may be communicated, and a multimedia data network through which multimedia data is communicated between multimedia components in the vehicle, and so on. For example, in-vehicle computing system 209 may retrieve from the engine CAN bus the current speed of the vehicle estimated by the wheels sensors, a power state of the vehicle via a battery and/or power distribution system of the vehicle, an ignition state of the vehicle, a condition of one or more air bags of the vehicle, a condition of hazard lights of the vehicle, a condition of the power source 204 described with respect to FIG. 2, and so on. In addition, other interfacing means such as Ethernet may be used as well without departing from the scope of this disclosure.

A storage device 308 may be included in the in-vehicle computing system 209 to store data such as instructions executable by operating system processor 314 and/or interface processor 320 in non-volatile form. Storage device 308 may store application data to enable in-vehicle computing system 209 to run an application for connecting to a cloud-based server and/or collecting information for transmission to the cloud-based server. The application may retrieve information gathered by vehicle systems/sensors, input devices (e.g., a user interface 318), data stored in one or more storage devices, such as a volatile memory 319A or a non-volatile memory 319B, devices in communication with the in-vehicle computing system, and so on. In-vehicle computing system 209 may further include a volatile memory 319A. Volatile memory 319A may be random access memory (RAM). Non-transitory storage devices, such as non-volatile storage device 208 and/or non-volatile memory 319B (e.g., non-transitory memory), may store instructions and/or code that, when executed by a processor (e.g., operating system processor 314 and/or interface processor 320), controls in-vehicle computing system 209 to perform one or more of the actions described in this disclosure.

One or more additional sensors may be included in a sensor subsystem 310 of in-vehicle computing system 209. For example, sensor subsystem 310 may include a camera, such as a rear view camera for assisting a user in parking the vehicle and/or a cabin camera for identifying a user (e.g., using facial recognition and/or user gestures). Sensor subsystem 310 of in-vehicle computing system 209 may communicate with and receive inputs from various vehicle sensors and may further receive user inputs. For example, the inputs received by sensor subsystem 310 may include transmission gear position, transmission clutch position, gas pedal input, brake pedal input, transmission selector position, vehicle speed, engine speed, mass airflow through the engine, ambient temperature, intake air temperature, and so on, as well as inputs from climate control system sensors (such as heat transfer fluid temperature, antifreeze temperature, fan speed, passenger compartment temperature , desired passenger compartment temperature, ambient humidity, and so on), an audio sensor detecting voice commands issued by a user, a fob sensor receiving commands from and optionally tracking the geographic location/proximity of a fob of the vehicle, and so on.

While certain vehicle system sensors may communicate with sensor subsystem 310 alone, other sensors may communicate with both sensor subsystem 310 and vehicle control system 330, or may communicate with sensor subsystem 310 indirectly via vehicle control system 330. A navigation subsystem 311 of in-vehicle computing system 209 may generate, transmit, receive, and/or process navigation information such as location information (e.g., via a GPS sensor and/or other sensors from sensor subsystem 310), route guidance, traffic information, point-of-interest (POI) identification, and/or provide other navigational services for the driver.

Vehicle control system 330 may include controls for controlling aspects of various vehicle systems 331 involved in different in-vehicle functions. These may include, for example, controlling aspects of vehicle audio system 332, aspects of a climate control system 334, aspects of a telecommunication system 336, and so on.

Vehicle control system 330 may also include controls for adjusting the settings of various vehicle control elements 361 (or vehicle controls, or vehicle system control elements) related to the engine and/or auxiliary elements within the cabin of the vehicle, such as one or more steering wheel controls 362 (e.g., steering wheel-mounted audio system controls, cruise controls, windshield wiper controls, headlight controls, turn signal controls, and so on), instrument panel controls, microphone(s), accelerator/brake/clutch pedals, a gear shift, door/window controls positioned in a driver or passenger door, seat controls, cabin light controls, audio system controls, cabin temperature controls, and so on. Vehicle control elements 351 may also include internal engine and vehicle operation controls (e.g., engine controller module, actuators, valves, and so on) that are configured to receive instructions via the CAN bus of the vehicle to change operation of one or more of the engine, exhaust system, transmission, and/or other vehicle system.

One or more elements of in-vehicle computing system 209 may be controlled by a user via user interface 318. User interface 318 may include a graphical user interface presented on a touch screen, such as touch screen 208 and/or display screen 211 of FIG. 2, and/or user-actuated buttons, switches, knobs, dials, sliders, and so on. For example, user-actuated elements may include steering wheel controls, door and/or window controls, instrument panel controls, audio system settings, climate control system settings, and the like. A user may also interact with one or more applications of in-vehicle computing system 209 via user interface 318. In addition to receiving a user's vehicle setting preferences on user interface 318. Notifications and other messages (e.g., received messages), as well as navigational assistance, may be displayed to the user on a display of the user interface. User preferences/information and/or response to presented messages may be performed via user input to the user interface.

Although the electronic controller 212 is shown including the operating system processor 314, memory 319A, memory 319B, and so on, in some embodiments the electronic controller 212 may include a different number and/or configuration of components. For example, the electronic controller 212 may additionally be integrated with the one or more antennas 306, the one or more transmitters 338, and so on.

Turning now to FIG. 4, a schematic block diagram of a respiratory characteristic estimation model 400 is shown. The respiratory characteristic estimation model 400 may be similar to the respiratory characteristic estimation model 116 described with respect to FIG. 1. As shown in FIG. 4, the respiratory characteristic estimation model 400 includes a first breathing wave convolutional feature extractor and a second convolutional module followed by a fully connected layers

For example, optical flow data 402, being represented as a 2D matrix, for example output from a sparse motion analysis, may be inputted into the respiratory characteristic estimation model 400, maintaining the shape of [number of analyzed points x 2, number of frames in the analyzed time window], where the first dimension can be interpreted as a number of channels optical flow signal for further convolution. The optical flow data 402 may be provided as input to breathing wave estimator 404, which is one dimensional convolution U-Net architecture. The breathing wave estimator 404, which may comprise a plurality of convolutions 406, thereby making up the first convolutional module of the architecture. The breathing wave estimator 404 may be configured to extract from the optical flow data 402 a breathing wave 412. The optical flow data 402 may also be taken to determine a mean optical flow 410 according to axis 408.

The breathing wave 412 and the mean optical flow 410 may be stacked along a convolutional channel dimension, increasing the channels number, according to axis 414. The combination of the breathing wave 412 and the mean optical flow 410 may result in the combined breathing wave + mean optical flow tensor 416. The purpose of merging the estimated breathing wave with mean optical flow is to convey the part of the original motion signal to the uncertainty part of the network, If characteristics of mean optical flow are drastically different comparing to estimated breathing wave, this is a key feature allowing the model to increase the uncertainty estimates.

The combined tensor 416 may be provided as input to the second convolutional layer. For example, the combined tensor 416 may be provided as input to a respiratory characteristic estimator 418. The respiratory characteristic estimator 418 may be particular to a specific characteristic, like breathing rate, or may be configured to generate estimations of more than one characteristic, like both breathing rate and breathing rate variability, making the architecture multi-head. For the purposes of this example, the respiratory characteristic estimator 418 may be a breathing rate estimator. The breathing rate estimator may comprise a plurality of convolutions 420, a flatten process 422, and one or more fully connected layers 422. An output of the plurality of convolutions 420 may be flatted by the flatten process 422. A flattened output (e.g., one-dimensional vector) may be inputted into the fully connected layers and the fully connected layers may then map the flatted output to an overall output, such as breathing rate quantities 426. Other overall outputs may be determined in other examples, such as breathing rate variability quantities in the example of a respiratory characteristic estimator intended for breathing rate variability estimation.

The respiratory characteristic estimation model 400 as herein disclosed may thus be a multi-modal model that ingests a single input and outputs both breathing wave and respiratory characteristic (e.g., breathing rate, breathing rate variability breathing phase, etc.) outputs. This may increase the overall interpretability and training stability of the model compared to models trained to only do one or the other.

Turning now to FIG. 5, a high-level flowchart illustrating a method 500 for respiratory characteristic estimation is shown. The method 500 may be executed by one or more processors of a vehicle computing system, such as processor(s) 104 of vehicle computing system 102 of FIG. 1. Some operations of the method 500 may be stored in a non-transitory memory of the vehicle computing system (e.g., non-transitory memory 106) and executed by the processor(s) of the vehicle computing system. In various embodiments, a respiratory characteristic estimation model, as referenced in the method 500, may be trained as will be described below.

Method 500 begins at 502, wherein method 500 includes obtaining in-vehicle visual signals. As described with respect to FIG. 1, the vehicle may be equipped with one or more visual sensing devices. In some examples, the visual sensing devices may be NIR cameras adapted to obtain images of a driver of the vehicle. For example, the NIR cameras may capture images at a predefined framerate, such as 30 fps. The images captured by the visual sensing devices may be transmitted to the vehicle computing system for processing.

At 504, method 500 includes extracting respiratory signals based on the visual signals. Extracting respiratory signals may include extracting motion data for given ROIs. As will be further described with respect to FIG. 6, extracting the respiratory signals may comprise ROI detection and motion analysis of the detected ROIs.

At 506, method 500 includes estimating respiratory characteristics based on the respiratory signals. In some examples, the respiratory characteristics may be quantities, such as is the case for a breathing rate characteristic, or labels, such as is the case for a breathing phase characteristic. The respiratory characteristics may be estimated by deployment of a trained respiratory characteristic estimation model, such as the respiratory characteristic estimation model 400 described with respect to FIG. 4. The respiratory characteristic estimation model may be trained to ingest respiratory signals (e.g., optical flow data) and output respiratory characteristics, in a chosen form (e.g., breathing rate quantities) according to a method described with respect to FIG. 7.

In some examples, estimation of uncertainty may be integrated into the architecture of the respiratory characteristic estimation model. In such examples, method 500 may additionally include estimating uncertainty in the estimated respiratory characteristics, as will be further described with respect to FIG. 8. The model estimates uncertainty in regression characteristics of breathing by employing a deep quantile regression paradigm. This approach facilitates the estimation of IQR based on the predicted quantiles of the breathing rate, serving as an integral component of the model. A post-processing step translates the IQR into uncertainty estimation, ensuring easy interpretability. This step represents the expected accuracy of the model and is achieved using a lookup table. The table maps the corresponding uncertainty to accuracy, derived from post-hoc analysis conducted on a validation dataset.

At 510, method 500 includes outputting respiratory characteristics, and corresponding uncertainty in examples in which uncertainty estimation is integrated into the respiratory characteristic estimation model. The respiratory characteristics, as outputted, may be transmitted to other vehicle systems for additional processing, in some examples. For example, the respiratory characteristics may be transmitted to a health and wellbeing system (e.g., health and wellbeing system 136), which may, as a non-limiting example, compare the respiratory characteristics to the driver's baseline in order to determine overall state of the driver. For example, a slower breathing rate compared to the driver's normal may indicate that the driver is sleepy. Such a determination may be used for downstream effects, such as adjustments in vehicle operation. For example, a sleepy driver state may result in adjustment of ADAS system operation, whereby, as a non-limiting example, autonomous driving may be enabled with the purpose of pulling the vehicle over to the side of a road, and a notification may be outputted to the driver indicating the reasoning and the action taken.

Turning now to FIG. 6, a flowchart illustrating a method for determining respiratory characteristics is shown. The method 600 may be executed by one or more processors of a vehicle computing system, such as processor(s) 104 of vehicle computing system 102 of FIG. 1. Some operations of the method 600 may be stored in a non-transitory memory of the vehicle computing system (e.g., non-transitory memory 106) and executed by the processor(s) of the vehicle computing system. In various embodiments, a respiratory characteristic estimation model, as referenced in the method 600, may be trained as will be described below.

At 602, method 600 includes capturing video data with one or more visual sensing devices. As described above, the vehicle may be equipped with one or more visual sensing devices, such as NIR cameras. These visual sensing devices may capture video data iteratively, for example at a predefined framerate.

At 604, method 600 includes identifying ROIs in video data. In some examples, frames of the video data may be processed to enhance contrast and decrease noise. Then, a subject's (e.g., a driver's) face, chest, and/or abdomen areas are identified and isolated. As an example, the subject's face, chest, and/or abdomen areas may be identified within a first frame of the video data. A face ROI, a chest ROI and/or an abdomen ROI may be identified and isolated by a segmentation or other object detection algorithm, such as a deep-learning-based segmentation approach, thresholding-based segmentation algorithm, an edge-based segmentation algorithm, a region-based segmentation algorithm, or a pose estimation algorithm. For example, with a thresholding-based segmentation algorithm, increasing the contrast prior to application of the algorithm may increase the accuracy of the segmentation. As another example, a pose estimation model can be used to spot the occlusion of the ROI by hands kept on the steering wheel and eliminate occluded key points for sparse motion analysis.

At 606, method 600 includes analyzing motion in the identified ROIs to determine movement data. Analyzing motion in the identified ROIs may include adaptively adjusting the ROIs, as noted at 608. For example, an algorithm that dynamically adjusts the ROI based on the subject's movements. Such movements of the subject may include posture changes, shifting in seat, leaning side to side or forward/back, twisting at the waist, for example when turning the steering wheel, reaching an arm out, and the like. An exemplary algorithm may be tracking the Intersection Over Union (IOU) metric between an anchor ROI and the current ROI estimated from face or body position. If the ROI is smaller than a predefined threshold, the algorithm collects motion from new region and the new motion is used for further processing.

Further, analyzing the motion in the ROIs may include applying a sparse motion analysis, as noted at 610. The sparse motion analysis may include an optical flow analysis method, in some examples, though it should be understood that other methods have been contemplated. For example, the optical flow motion analysis may estimate the motion vector field in consecutive frames of the video data, thereby indicating how pixels shift between frames. In some examples, the optical flow analysis may be applied to specific portions of the ROIs, such as corners, edges, or blobs (e.g., groups of pixels) rather than estimating motion for every pixel, thereby reducing overall processing demands on the system.

At 608, method 600 includes determining breathing wave and respiratory characteristics based on the movement data. Breathing wave and respiratory characteristics may be determined by deploying a trained respiratory characteristic estimation model, as noted at 614, as described herein. The trained respiratory characteristic estimation model may be trained to determine both the breathing wave and one or more selected respiratory characteristics. For example, the exemplary respiratory characteristic estimation model shown in FIG. 4 specifies breathing rate as the respiratory characteristic, though it should be understood that another characteristic or combination of characteristics, such as breathing rate variability or breathing phase, may also be determined.

The output of the trained respiratory characteristic estimation model may be a breathing wave and annotated respiratory characteristics. In examples in which uncertainty estimation is integrated into the respiratory characteristic estimation model, the model may additionally output uncertainty predictions. Determination of uncertainty will be further described with respect to FIG. 8.

Separating estimation of respiratory characteristics from extracting motion data may reduce the overall size of the neural network, thereby increasing its processing efficiency and allows for repurposing of the sparse motion analysis/optical flow analysis algorithms from other pipelines. For example, the ADAS may utilize a sparse motion analysis for other purposes, such as detection and tracking of other vehicles on the road. This same sparse motion analysis algorithm may be repurposed by the vehicle computing system to extract motion data from video data. In this way, required memory storage may be reduced. Separating the preprocessing (e.g., extraction of motion data) from the estimation model may also allow for easy parallelization on GPUs and neural processing units (NPUs).

At 616, method 600 includes outputting the breathing wave and the respiratory characteristics. For example, respiratory characteristic estimates may be provided to both a infotainment system (e.g., infotainment unit 132 of FIG. 1), a health and wellbeing system (e.g., health and wellbeing system 136 of FIG. 1), and to an ADAS system (e.g., ADAS system 134 of FIG. ) of the vehicle. As is described above, the health and wellbeing system may further process the respiratory characteristics, for example for analysis of driver state (e.g., stress level, alertness, cognitive load, and the like).

Additionally, the ADAS system may adjust one or more parameters (e.g., operating states) of the vehicle in response to the respiratory characteristics, as described above. When uncertainty is also estimated, high uncertainty predictions (e.g., uncertainty predictions above a predefined threshold), may be flagged for the system to address.

Turning now to FIG. 7, a flowchart illustrating a method 700 for training a respiratory characteristic estimation model is shown. The method 700 may be executed by one or more processors based on instructions stored in non-transitory memory. In some examples, the method 700 may be executed outside of the vehicle system (e.g., in a development system) and then once completed, the trained model may be downloaded as software onto a vehicle computing system of a vehicle.

At 702, method 700 includes collecting ground truth data. To capture ground truth data, method 700 includes capturing video data, as noted at 704, determining, based on the video data, ground truth motion data, as noted at 706, and determining a ground truth breathing wave, as noted at 708. Video data may be captured in a vehicle environment, for example during a real driving scenario or during a simulated driving scenario. The ground truth breathing wave may be generated by extracting motion data using similar techniques described herein, including ROI identification and sparse motion analysis, which generates the ground truth motion data. Based on the ground truth motion data, motion can be tracked to identify periodic oscillations corresponding to breathing motion. Once a cyclical pattern is identified, the amplitude of motion can be plotted over time. This plot may be the breathing wave. This breathing wave may be considered a "ground truth" breathing wave because it is determined by standard techniques rather than via an AI model and thus may be considered "true" in this context. The ground truth may be established for end points, such as breathing wave and breathing rate in this example, and other predicted parameters like motion vectors may be used as an intermediate step to predict breathing wave and breathing rate. In some examples, the in-vehicle sensor may output a rough, unprocessed, breathing wave signal, which may then be normalized to a range of (-1,1). The normalized breathing wave may be the ground truth breathing wave herein described.

At 710, method 700 includes semi-automatically converting the ground truth breathing wave to one or more chosen respiratory characteristics. Semi-automatically converting the ground truth breathing wave may include applying one or more automated scripts, as noted at 712, and obtaining human supervision conversions, as noted at 714. The automated pre-annotation of raw breathing wave signal identifies peaks and valleys for further human refinement and a binary flag denoting a signal reliability score (if 0, there is no use in annotating those fragments, since the signal does not exhibit any breathing patterns). The pre-annotation script significantly reduces processing time by enabling human annotators to focus only on correcting problematic signals, particularly those affected by noise. In addition, a complementary script evaluates the raw breathing wave signal to estimate the expected uncertainty in ground truth annotations produced by human annotators. This uncertainty estimation is generated using a linear model trained on a dataset annotated by multiple human participants to capture variance of the annotations. Consequently, each processed file includes the normalized breathing wave data, annotated peaks and valleys, and a quantified measure of expected variability in ground truth annotations for respiratory metrics, such as breathing rate.

Human supervision conversions may include human-performed annotations of the breathing wave, including portions of the signal with a low reliability score, as determined by the automated scrips. For example, breathing rate quantities may be annotated to those portions. Similarly, breathing phase (e.g., inhale vs exhale vs hold) may be annotated manually to those portions. Human annotations may be performed based on previous knowledge of the human expert who is looking at the generated breathing wave. Semi-automatically converting the ground truth breathing wave to the chosen respiratory characteristics may reduce the time needed to generate ground truth annotations by reducing the overall volume of data to be analyzed by human experts, thus allowing for a more robust training of the model.

The ground truth motion data, ground truth breathing waves, and annotated respiratory characteristics may correspond to a variety of breathing types, in some examples. For example, the ground truth may include data from healthy persons as well as from persons with various respiratory conditions, like asthma, chronic obstructive pulmonary disease, and chronic respiratory failure. Further ground truth data may be generated for various driving conditions, including frequently moving drivers, drivers interacting with passengers, for moments when drivers turn the steering wheel, reach their arms out, twist in their chair, and the like. The ground truth breathing waves and annotated respiratory characteristics may be provided in the context of those driving and movement conditions, thereby allowing the estimation model to be trained to determine respiratory characteristics even in such scenarios.

At 716, method 700 includes training the respiratory characteristic estimation model on the ground truth motion data and the annotated respiratory characteristics. More specifically, training the respiratory characteristic estimation model on the ground truth motion data and the annotated respiratory characteristics includes training the model to learn to map the ground truth data to a breathing wave and the breathing wave to one or more respiratory characteristics (e.g., breathing rate, breathing phase, etc.). In some embodiments, the model may comprise a U-net convolutional neural network. In some embodiments, the model may comprise a recurrent model architecture, Transformer-based architecture or State Space approach architecture.

The convolutional filters of the architecture may comprise a plurality of weights, wherein the values of the weights are learned during a training procedure. The convolutional filters may corresponding to one or more visual features/patters, thereby enabling the model to determine a breathing wave based on motion data extracted from visual video data of a ROI of a driver and to estimate, based on the breathing wave, the respiratory characteristics. In other embodiments, the respiratory characteristic estimation model may not be a convolutional neural network, rather may be a different type of network, for example recurrent or attention-based.

Training the respiratory characteristic estimation model on the ground truth motion data, the breathing wave ground truth, and the annotated respiratory characteristics may include iteratively inputting a ground truth motion dataset into a first input layer of the model. In some embodiments, each value of the inputted data may input into a distinct neuron of the first input layer of the model. The model may map the input data to a corresponding target breathing wave by propagating the input data from the first input layer, through one or more first hidden layers, until reaching a first output layer. This process may then be repeated, whereby the target breathing wave is inputted into a second input layer of the model, through one or more second hidden layers, until reaching a second output layer. In some embodiments, the output of the model may be respiratory characteristics (e.g., quantities, labels, other annotations, or the like) as well as the breathing wave. In this way, the model may be trained to ingest motion data, for example as extracted from video data as described with respect to FIGS. 4 and 5, and determine both a breathing wave and annotated respiratory characteristics.

The respiratory characteristic estimation model may be configured to iteratively adjust its weights of the model in order to minimize a loss function, based on an assessment of differences between the input and the target comprised by each pair of training pairs (or trio of training trios). In one embodiment, the loss function is a Quantile Regression Loss function for the regression head (e.g. for breathing rate quantiles prediction). For the breathing wave estimation, the loss function may be a negated Pearson correlation coefficient loss function, which compares predicted breathing wave and ground truth breathing wave, forcing proper signal monotonicity. It should be appreciated that the examples provided herein are for illustrative purposes, and other types of loss function may be used without departing from the scope of this disclosure.

The weights and biases of the model may be adjusted based on a difference between the output and the target (e.g., ground truth respiratory characteristic and/or breathing wave) of the relevant training pair (or training trio). The difference (or loss), as determined by the loss function, may be back propagated through the neural learning network to update the weights (and biases) of the convolutional layers. In some embodiments, back propagation of the loss may occur according to a gradient descent algorithm, wherein a gradient of the loss function (a first derivative, or approximation of the first derivative) is determined for each weight and bias of the deep neural network. Each weight (and bias) of the respiratory characteristic estimation model is then updated by adding the negative of the product of the gradient determined (or approximated) for the weight (or bias) with a predetermined step size. Updating of the weights and biases may be repeated until the weights and biases of the respiratory characteristic estimation model converge, or the rate of change of the weights and/or biases of the deep neural network for each iteration of weight adjustment are under a threshold.

In order to avoid overfitting, training of the respiratory characteristic estimation model may be periodically interrupted to validate a performance of the model on test pairs (or test trios). In an embodiment, training of the model may end when a performance of the model on the test pairs (or test trios) converges (e.g., when an error rate on the test set converges on or to within a threshold minimum value). In this way, the respiratory characteristic estimation model may be trained to generate a breathing wave based on extracted motion data of a driver and to generate chosen respiratory characteristics (e.g., breathing rate, breathing phase, etc.) based on the breathing wave.

In some embodiments, an assessment of the performance of the respiratory characteristic estimation model may include a combination of minimum error rate and a quality assessment, or a different function of the minimum error rates achieved on each pair or trio of the test pairs or trios and/or one or more quality assessments, or another factor for assessing the performance of the respiratory characteristic estimation model. It should be appreciated that the examples provided herein are for illustrative purposes, and other loss functions, error rates, quality assessments, and/or performance assessments may be included without departing from the scope of this disclosure.

Turning now to FIG. 8, a flowchart illustrating a method 800 for estimating uncertainty in a quantification based output of a respiratory characteristic estimation model is shown. In some examples, the method 800 may be executed by one or more processors according to instructions stored in non-transitory memory of a vehicle (e.g., by processor(s) 104 according to instructions stored in non-transitory memory 106 of the vehicle system 100 of FIG. 1). It should be understood that the method herein described for estimation of uncertainty, while described as being applied to the respiratory characteristic estimation model herein disclosed, may be applied to and/or integrated within other neural networks in other circumstances. As will be herein described, determination of uncertainty involves estimating aleatoric uncertainty using the IQR. The IQR is defined as the difference between the 90th quantile and the 10th quantile. After calculating the IQR based on the model outputs (quantified outputs), the IQR value may represent the expected range of the regression model. Subsequently, on a separate validation dataset, how the IQR range corresponds to the expected accuracy of the model is evaluated and a mapping from IQR range to the expected accuracy is determined. As a result, the final uncertainty measure is expressed in terms of expected accuracy, which is derived from the IQR.

At 802, method 800 includes obtaining breathing wave and annotated respiratory characteristics from the respiratory characteristic model. As is described with respect to FIGS. 5 and 6, one or more visual sensing devise (e.g., NIR cameras) within the vehicle may capture video data (e.g., comprising a plurality of frames). One or more relevant ROIs may be identified within the video data and motion data may be extracted from the video data for those ROIs. The motion data may be fed into the trained respiratory characteristic estimation model, which may output a breathing wave and annotated respiratory characteristics (for which the model is trained, such as breathing rate or breathing phase). In some examples, the annotated respiratory characteristics may be outputted as quantities. For example, for a breathing rate characteristic, the 10th percentile of breathing rate, the 50th percentile of breathing rate, and the 90th percentile of breathing rate may be outputted.

At 804, method 800 includes determining IQR based on the annotated respiratory characteristics. As an example where the characteristic is breathing rate, wherein the 10th, 50th, and 90th quantiles of breathing rate are outputted by the respiratory characteristic estimation model, IQR is calculated based on the 10th and 90th percentiles, for example according to equation (1):

$$IQR \, (10-90) = P_{90} - P_{10} \qquad\qquad (1)$$

where $IQR$ (10 - 90) is the interquartile range between the 10th quartile output and the 90th quartile output, $P_{90}$ is the 90th quartile output, and $P_{10}$ is the 10th quartile output.

At 806, method 800 includes determining, based on the IQR comparison, an uncertainty estimation. In some examples, the uncertainty estimation may be determined based on a quantile regression algorithm. In some examples, IQR can be directly converted into expected accuracy (e.g., ACC(3BPM) or ACC(5BPM)), which may allow for representation of model uncertainty in a relative 0-1 scale. ACC(3BPM) may represent the percentage of samples where the breathing rate estimator produces an error smaller than 3 BPM. Thus, when the model's error is less than 3 BPM, the model output may be considered accurate, and when the model's error is greater than 3 BPM, the model output may be considered inaccurate.

In some examples, the uncertainty estimation may be outputted along with the breathing wave and annotated respiratory characteristics from the respiratory characteristic estimation model. While the annotated respiratory characteristics may be outputted to various vehicle systems (e.g., a health and wellbeing system, an ADAS, etc.) when appropriate, the uncertainty estimation may be outputted to the vehicle computing system, flagged for additional processing to be handled appropriately. For example, a flag for additional processing may be a trigger for stopping other functionalities that depend on reliable predictions of the breathing estimation functionality. The uncertainty estimation may provide information about the reliability of the prediction from the respiratory characteristic estimation model. Thus, the outputs of the model may be considered in the light of the uncertainty estimation with regard to downstream actions.

For example, as at 808, method 800 includes determining if the uncertainty is greater than a threshold, like ACC(3BPM). When greater than the threshold, method 800 proceeds to 810 to transmit the breathing wave and annotated respiratory characteristics to the appropriate vehicle systems for processing. For example, the breathing wave and annotated respiratory characteristics may be transmitted to a health and wellbeing system (e.g., health and wellbeing system 136) or a drive monitoring system to be processed to determine human states like drowsy, alert, asleep, etc. which may then be used to affect vehicle systems like ADAS.

If the uncertainty is less than the threshold, method 800 proceeds to 812 to flat the annotated respiratory characteristics as unreliable. Then, these annotated respiratory characteristics may be filtered out, as noted at 814. In this way, these filtered out annotated respiratory characteristics may not be transmitted to vehicle systems like the health and wellbeing system and the driver monitoring systems. For example, in an instance when the model outputs a breathing rate below a predefined threshold for the health and wellbeing system and/or ADAS system, for which one or more actions, such as changing ADAS operation, would be taken, but the corresponding uncertainty estimation for that output is high, the system may take this into account and may not proceed to take action as the output may be considered unreliable.

Uncertainty estimation of outputs of the respiratory characteristic estimation model may be robust to unreliable motion data, such as in moments when external factors reduce the quality of the signal. For example, on bumpy roads, extracted motion data may not be reliably accurate to the driver's actual respiration. Uncertainty estimation may capture this external factor and may determine that an output of the estimation model is unreliable therefor. Other external factors, like motion artifacts, interference from other passengers, speech-related noise, and coughing or sneezing may also be captured by this uncertainty estimation.

Further, in some examples, data from other vehicle sensors may be incorporated into uncertainty estimation. For example, motion artifact as a result of the driver turning a steering wheel, whereby the driver's arms temporarily cross over the chest region may be confirmed from cross-reference to steering wheel sensor data. As another example, activation of a setting such as traction control may be used to contextualize uncertainty estimation, for example in scenarios in which road surface may affect motion data signals.

FIG. 9 shows a graph 900 plotting various breathing rate quantities demonstrating output of the trained respiratory characteristic estimation model and estimated uncertainty therein. For example, a first trend 902 may

**EP 4 760 658 A1**

represent the outputted breathing rate from the trained respiratory characteristic estimation model for a given inputted motion dataset. A second trend 904 may represent a ground truth breathing rate for the same motion dataset. A third trend 906 (e.g., a shaded area around the first and/or second trends 902, 904) may indicate a calculated IQR based on the 90th and 10th percentiles of breathing rate as outputted by the respiratory characteristic estimation model. A fourth trend 908 may indicate a quality of the ground truth data.

The quality of the ground truth data may be quantified as the various between BPM, derived from an annotation process (e.g., a semi-automatic annotation process as previously discussed). A higher curve of the fourth trend 98 may indicate a higher various in the ground truth data, suggesting that the ground truth data is less reliable in these instances. When the variance is higher (e.g., when the values of the fourth trend 908 are higher), the model's performance tends to degrade, following higher uncertainty in its predictions. Thus, when the values of the fourth trend 908 are higher, the size of the third trend 906 (e.g., the width of the shaded area) may be greater, thus indicating a higher IQR value.

As a non-limiting example, in several parts of the graph 900, the fourth trend 908 remains flat at the level of 20 BPM. This flat line indicates sections where the reliability score is 0, meaning these data points were not annotated at all. As a result, no various or accuracy information could be derived from the annotation process. In such scenarios, the uncertainty estimation may be the highest possible uncertainty, indicating its reduced confidence in making predictions on unseen or poor-quality data.

In contrast, the initial portion of the graph 900 (e.g., from 0 to 7000 frames) shows a period where the uncertainty is relatively low, as indicated by the relatively narrow width of the third trend 906, which is presumed to be correlated with ground truth data of high quality. During this period, the quality of the annotations is high, as indicated by the low value of the fourth trend 908. This may suggest that the model exhibits a low level of uncertainty when faced with low-variance annotations and good input data quality. Overall, the graph 900 demonstrates the correlation between IQR, as indicated by the third trend 906, and the quality of the ground truth data, thus suggesting that IQR is a parameter that can be used for estimation of uncertainty, as is herein presented.

Of note, the uncertainty level of the estimation model may remain consistent regardless of the breathing rate level. Whether the BPM is low or high, relatively, the uncertainty can still remain low, indicating that the model is capable of accurately predicting both low and high breathing rates, as an exemplary respiratory characteristic. Therefore, the estimation model may not be biased towards any particular range of a given characteristic and may maintain a stable confidence in its predictions across varying breathing speeds.

The technical effect of the systems and methods herein provided is that respiratory characteristics and uncertainty thereof may be robustly estimated in a vehicular environment in a non-invasive manner. The systems and methods herein combine advanced computer vision techniques with specially trained deep learning architectures to provide reliable in-cabin respiratory characteristic estimation. Training the estimation model (e.g., a convolutional neural network) on a variety of ground truth data that captures various driving conditions, including various health conditions of the drivers and movement scenarios of the drivers, the model may more accurately estimate respiratory conditions. Further, uncertainty estimation may capture unreliable data and thus unreliable model outputs in an efficient manner.

The disclosure also provides support for a vehicle computing system of a vehicle, comprising: one or more processors, memory storing instructions that when executed cause the one or more processors to: obtain video data comprising a plurality of frames from one or more visual sensing devices within the vehicle, extract motion data from the video data, determine, from the motion data, a breathing wave and one or more respiratory characteristics of a subject within the vehicle, and output the breathing wave and one or more respiratory characteristics to one or more vehicle systems. In a first example of the system to extract motion data from the video data, the one or more processors are configured to: identify, within a first frame of the plurality of frames, one or more regions of interest (ROIs), dynamically adjust the one or more ROIs based on the subject's movements, and perform sparse motion analysis to the one or more ROIs. In a second example of the system, optionally including the first example, the sparse motion analysis includes an optical flow analysis. In a third example of the system, optionally including one or both of the first and second examples to determine, from the motion data, the breathing wave and the one or more respiratory characteristics, the one or more processors are configured to deploy a respiratory characteristic estimation model trained to determine breathing wave and the one or more respiratory characteristics from motion data. In a fourth example of the system, optionally including one or more or each of the first through third examples, the respiratory characteristic estimation model is a convolutional neural network. In a fifth example of the system, optionally including one or more or each of the first through fourth examples, the respiratory characteristic estimation model is trained based on ground truth breathing wave and respiratory characteristics that are semi-automatically converted from the ground truth breathing wave based on a combination of automated scripts and human supervised annotations. In a sixth example of the system, optionally including one or more or each of the first through fifth examples, the memory stores further instructions that when executed cause the one or more processors to estimate uncertainty in the breathing wave and one or more respiratory characteristics. In a seventh example of the system, optionally including one or more or each of the first through sixth examples to estimate uncertainty for quantification based model outputs, the one or more processors are configured to determine an interquartile range (IQR) of quantities of the one or more respiratory characteristics and determine, based on the IQR, an accuracy of the one or more respiratory characteristics.

The disclosure also provides support for a method for training a respiratory characteristic estimation model, comprising: capturing video data, determining, from the video data, ground truth motion data, determining, based on the ground truth motion data, ground truth breathing waves, semi-automatically converting the ground truth breathing waves to one or more annotated respiratory characteristics, training the respiratory characteristic estimation model on training trios of the ground truth motion data, the ground truth breathing waves, and the one or more annotated respiratory characteristics. In a first example of the method, semi-automatically converting the ground truth breathing waves to one or more annotated respiratory characteristics comprises applying one or more automated scripts to the ground truth breathing waves. In a second example of the method, optionally including the first example, applying the one or more automated scripts comprises identifying peaks and valleys within the ground truth breathing wave and a binary flat denoting signal reliability scores. In a third example of the method, optionally including one or both of the first and second examples, semi-automatically converting the ground truth breathing waves further comprises receiving human supervised annotations to one or more portions of the ground truth breathing waves based on the signal reliability scores. In a fourth example of the method, optionally including one or more or each of the first through third examples, the trained respiratory characteristic estimation model is configured for deployment to determine, from visual signals, a breathing wave and one or more corresponding respiratory characteristics. In a fifth example of the method, optionally including one or more or each of the first through fourth examples, the one or more annotated respiratory characteristics comprise one or more of breathing rate, breathing phase, and breathing rate variability.

The disclosure also provides support for a method, comprising: obtaining in-vehicle video data with an in-vehicle camera, wherein the in-vehicle video data comprises a plurality of frames, extracting respiratory signals from the in-vehicle video data, estimating respiratory characteristics with a respiratory characteristic estimation model, and outputting the estimated respiratory characteristics to one or more vehicle systems. In a first example of the method, extracting, from the in-vehicle video data, respiratory signals comprises: identifying one or more ROIs within the video data, and analyzing motion of the one or more ROIs to determine the respiratory signals, wherein the respiratory signals are ground truth motion data. In a second example of the method, optionally including the first example, identifying one or more ROIs comprises applying an object detection algorithm to a first frame of the in-vehicle video data and then adaptively adjusting the one or more ROIs in subsequent frames based on motion vectors. In a third example of the method, optionally including one or both of the first and second examples, the method further comprises: determining uncertainty of the estimated respiratory characteristics based on interquartile range of outputs of the respiratory characteristic estimation model. In a fourth example of the method, optionally including one or more or each of the first through third examples when uncertainty is below a predefined threshold, outputting the estimated respiratory characteristics comprises outputting the estimated respiratory characteristics to one or more of a health and wellbeing system and an advanced driver assistance system. In a fifth example of the method, optionally including one or more or each of the first through fourth examples when uncertainty is above a predefined threshold, outputting the estimated respiratory characteristics comprises flagging the estimated respiratory characteristics as unreliable.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A vehicle computing system of a vehicle, comprising:

    one or more processors;
    memory storing instructions that when executed cause the one or more processors to:

obtain video data comprising a plurality of frames from one or more visual sensing devices within the vehicle;
extract motion data from the video data;
determine, from the motion data, a breathing wave and one or more respiratory characteristics of a subject within the vehicle; and
output the breathing wave and one or more respiratory characteristics to one or more vehicle systems.

2. The vehicle computing system of claim 1, wherein, to extract motion data from the video data, the one or more processors are configured to:

identify, within a first frame of the plurality of frames, one or more regions of interest (ROIs);
dynamically adjust the one or more ROIs based on the subject's movements; and
perform sparse motion analysis to the one or more ROIs.

3. The vehicle computing system of claim 2, wherein the sparse motion analysis includes an optical flow analysis.

4. The vehicle computing system of claim 1, wherein, to determine, from the motion data, the breathing wave and the one or more respiratory characteristics, the one or more processors are configured to deploy a respiratory characteristic estimation model trained to determine breathing wave and the one or more respiratory characteristics from motion data.

5. The vehicle computing system of claim 4, wherein the respiratory characteristic estimation model is a convolutional neural network.

6. The vehicle computing system of claim 4, wherein the respiratory characteristic estimation model is trained based on ground truth breathing wave and respiratory characteristics that are semi-automatically converted from the ground truth breathing wave based on a combination of automated scripts and human supervised annotations.

7. The vehicle computing system of claim 1, wherein the memory stores further instructions that when executed cause the one or more processors to estimate uncertainty in the breathing wave and one or more respiratory characteristics.

8. The vehicle computing system of claim 7, wherein, to estimate uncertainty for quantification based model outputs, the one or more processors are configured to determine an interquartile range (IQR) of quantities of the one or more respiratory characteristics and determine, based on the IQR, an accuracy of the one or more respiratory characteristics.

9. A method for training a respiratory characteristic estimation model, comprising:

capturing video data;
determining, from the video data, ground truth motion data;
determining, based on the ground truth motion data, ground truth breathing waves;
semi-automatically converting the ground truth breathing waves to one or more annotated respiratory characteristics;
training the respiratory characteristic estimation model on training trios of the ground truth motion data, the ground truth breathing waves, and the one or more annotated respiratory characteristics.

10. The method of claim 9, wherein semi-automatically converting the ground truth breathing waves to one or more annotated respiratory characteristics comprises applying one or more automated scripts to the ground truth breathing waves.

11. The method of claim 10, wherein applying the one or more automated scripts comprises identifying peaks and valleys within the ground truth breathing wave and a binary flat denoting signal reliability scores.

12. The method of claim 11, wherein semi-automatically converting the ground truth breathing waves further comprises receiving human supervised annotations to one or more portions of the ground truth breathing waves based on the signal reliability scores.

13. The method of claim 9, wherein the trained respiratory characteristic estimation model is configured for deployment to determine, from visual signals, a breathing wave and one or more corresponding respiratory characteristics.

14. The method of claim 9, wherein the one or more annotated respiratory characteristics comprise one or more of breathing rate, breathing phase, and breathing rate variability.

100

VEHICLE COMPUTING SYSTEM 102

PROCESSOR(S) 104

NON-TRANSITORY MEMORY 106

VIDEO PROCESSING MODULE 108

ROI DETECTOR 110

ADAPTIVE ROI SELECTOR 112

MOTION ANALYSIS MODULE 114

ESTIMATION MODEL 116

TRAINING MODULE 118

UNCERTAINTY ESTIMATION MODEL 120

VISUAL SENSING DEVICE(S) 130

INFOTAINMENT UNIT 132

ADAS SYSTEM 134

HEALTH AND WELLBEING SYSTEM 136

# FIG. 1

**FIG. 2**

FIG. 3

**IN-VEHICLE COMPUTING SYSTEM 209**

MICROPHONE 302

SENSOR SUBSYSTEM 310
CAMERA(S)

USER INTERFACE 318
TOUCH SCREEN 208
DISPLAY SCREEN

**ELECTRONIC CONTROLLER 212**

OPERATING SYSTEM PROCESSOR 314

STORAGE DEVICE 308
APP(S)

SPEECH PROCESSING UNIT 304

NAVIGATION SUBSYSTEM 311

MEMORY 319A

MEMORY 319B

INTERFACE PROCESSOR 320
INTER-VEHICLE SYSTEM COMMUNICATION MODULE 322

ANTENNAS 306

TRANSMITTERS 338

COMMUNICATIONS SYSTEM 312

202

EXTERNAL DEVICES 250

**VEHICLE CONTROL SYSTEM 330**

VEHICLE SYSTEM 331
AUDIO SYSTEM 332
CLIMATE CONTROL SYS. 334
TELECOMM. SYS. 336

VEHICLE CONTROLS 361
STEERING CONTROL 362

**FIG. 4**

START

500

502

OBTAIN IN-VEHICLE VISUAL SIGNALS

504

EXTRACT RESPIRATORY SIGNAL BASED ON THE VISUAL SIGNALS (SEE FIG. 6)

506

ESTIMATE RESPIRATORY CHARACTERISTICS BASED ON RESPIRATORY SIGNAL

ESTIMATE UNCERTAINTY IN ESTIMATED RESPIRATORY CHARACTERISTICS (SEE FIG. 8)
508

510

OUTPUT RESPIRATORY CHARACTERISTICS AND CORRESPONDING UNCERTAINTY

END

# FIG. 5

FIG. 6

700

START

702

COLLECT GROUND TRUTH DATA

CAPTURE VIDEO DATA 704

DETERMINE GROUND TRUTH MOTION DATA 706

DETERMINE GROUND TRUTH BREATHING WAVE 708

710

SEMI-AUTOMATICALLY CONVERT GROUND TRUTH BREATHING WAVE TO CHOSEN RESPIRATORY CHARACTERISTIC(S)

APPLY AUTOMATED SCRIPTS 712

OBTAIN HUMAN SUPERVISION ANNOTATIONS 714

716

TRAIN RESPIRATORY CHARACTERISTIC MODEL ON GROUND TRUTH MOTION DATA, GROUND TRUTH BREATIHNG WAVES, AND ANNOTATED CHOSEN RESPIRATORY CHARACTERISTIC

END

# FIG. 7

FIG. 8

FIG. 9

| | Europäisches Patentamt European Patent Office Office européen des brevets | **EUROPEAN SEARCH REPORT** | **Application Number** EP 25 21 9089 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GUO KAIWEN ET AL: "Contactless Vital Sign Monitoring System for In-Vehicle Driver Monitoring Using a Near-Infrared Time-of-Flight Camera", APPLIED SCIENCES, vol. 12, no. 9, 27 April 2022 (2022-04-27), pages 1-23, XP093388537, DOI: 10.3390/app12094416 | 1-3,7,8 | INV. G06V20/59 G06V10/82 ADD. G06V10/25 G06V10/774 G06V10/776 |
| A | * page 5, paragraph 1 - page 6, last paragraph; figures 1-3, 5; table 1 * * page 8, paragraph 3 * ----- | 4-6,9-14 | |
| A | XIN LIU ET AL: "Multi-Task Temporal Shift Attention Networks for On-Device Contactless Vitals Measurement", ARXIV.ORG CORNELL UNIVERSITY LIBRARY, 201 OLIN LIBRARY CORNELL UNIVERSITY ITHACA, NY 14853, 28 February 2021 (2021-02-28), pages 1-6, XP081885840, * page 6, paragraph 1 - page 7, paragraph 3; figure 2; table 1 * * page 8, paragraph 5 * * equations (9)-(12); page 12, last paragraph - page 13, paragraph 10 * ----- | 9-14 | TECHNICAL FIELDS SEARCHED (IPC) G06V G06F |

-/--

The present search report has been drawn up for all claims

| Place of search | Date of completion of the search | Examiner |
|---|---|---|
| The Hague | 29 April 2026 | Granger, Bruno |

Europäisches
Patentamt

European
Patent Office

Office européen
des brevets

**EUROPEAN SEARCH REPORT**

Application Number

EP 25 21 9089

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BRENNAN AARON ET AL: "Respiration Rate Detection for In-Cabin Passenger Monitoring", 2023 34TH IRISH SIGNALS AND SYSTEMS CONFERENCE (ISSC) IEEE, 13 June 2023 (2023-06-13), pages 1-6, XP034368402, DOI: 10.1109/ISSC59246.2023.10162104 [retrieved on 2023-07-03] * abstract * * page 3, left-hand column, paragraph 1 - page 4, left-hand column, paragraph 4; figures 1-3 * * page 4, right-hand column, paragraph 1 - paragraph 2; table 1 * | 9-14 | |

-----

TECHNICAL FIELDS
SEARCHED      (IPC)

The present search report has been drawn up for all claims

| Place of search | Date of completion of the search | Examiner |
|---|---|---|
| The Hague | 29 April 2026 | Granger, Bruno |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................

& : member of the same patent family, corresponding
    document

EPO FORM 1503 03.82 (P04C01)